# EUROPEAN PATENT APPLICATION

(11) **EP 3 731 153 A1**
(43) Date of publication of application: **28.10.2020**
(21) Application number: 19195582.2
(22) Date of filing: 05.09.2019
(51) Int. Cl.: G06N 3/08

(54) **CLOUD-BASED TRANSACTION SYSTEM AND METHOD CAPABLE OF PROVIDING NEURAL NETWORK TRAINING MODEL IN SUPERVISED STATE**

(30) Priority: 26.04.2019 TW 108114677; 02.07.2019 TW 108123294
(71) Applicant: Ever Fortune.AI Co., Ltd., Taichung City 403 (TW)
(72) Inventor: HUANG, Tzung-Chi, Taichung City 403 (TW); LIAO, Ken Ying-Kai, Taichung City 403 (TW); TSAI, Fuu-Jen, Taichung City 403 (TW)
(74) Representative: Straus, Alexander

(57) **Abstract**

A cloud-based transaction system (10)(10') and method capable of providing a neural network training model in a supervised state perform training with different training programs on a plurality of pieces of data (101)(101') and thus obtain different training models to not only allow a client (201)(201') to conduct transactions on a third-party transaction platform (702) from a remote end but also allow the client (201)(201') to download the different training models according to transaction results under the supervision of a supervision unit (600)(600') to allow the client (201)(201') to compare the accuracy of the different training models, thereby enhancing the accuracy of the training models.

## Description

### BACKGROUND OF THE INVENTION

### 1. Technical Field

The present disclosure relates to technology of cloud-based transactions pertaining to neural network training models and, more particularly, to a cloud-based transaction system and method capable of providing a neural network training model in a supervised state, so as to allow users to use training data in a graphical medical big data database freely and conduct paid transactions.

### 2. Description of Related Art

The intertwined stacking relationships among artificial intelligence, machine learning, and neural network deep learning determine how accurate they are in functional predictions in various fields. Deep learning, the most primary one of these, is the keystone of implementation of machine learning, so as to simulate human intelligence. To make highly accurate functional predictions, deep learning entails performing algorithmic feature learning on data in a sophisticated neural network. Furthermore, the prior art does not disclose any integral, complete, big data-based, precise graphical medical database available to artificial intelligence talents worldwide; as a result, this poses a bottleneck to the development and application of medical artificial intelligence. In addition, when in possession of an integral, complete, big data-based, precise graphical medical database, enterprises, institutions, organizations and government agencies are reluctant to render these data accessible to the public but keen on rendering these data dedicated to their exclusive use, research and development. However, even though the enterprises, institutions, organizations and government agencies are in possession of an integral, complete, big data-based, precise medical image annotation database, there are no talents to develop a new algorithm for training high-precision models and high-precision applications. The lack of talents not only leaves little room for improvement in model accuracy but also leads to narrow scope of application.

The technology of training a neural network on a piece of data to create a model is well known among. For example, Taiwan patent 1645303 discloses (in claim 18) training a neural network on a feature data. Furthermore, Taiwan patent 1662511 discloses (in claims 5, 6) training different deep convolutional neural networks (DCNN) to create different classification models.

Therefore, the inventor of the present disclosure believes that products should be ever improving and thus endeavors to conduct related research, taking advantage of the inventor's years of experience in product design and development. Finally, the inventor puts forth the present disclosure.

### BRIEF SUMMARY OF THE INVENTION

Conventional graphical medical big data databases do not allow users to access a training model, which results from neural network training, with a paid transaction mechanism, and thus the training model does not have any room for improvement in accuracy.

To overcome the aforesaid drawback of the prior art, the present disclosure provides a cloud-based transaction system and method capable of providing a neural network training model in a supervised state to provide access to a graphical medical big data database, provide a trained pre-training model, and provide a training program whereby the users obtain a post-training model which results from training carried out with the training program, thereby informing the users of differences in accuracy between different training models.

In order to achieve the above and other objectives, the present disclosure provides a cloud-based transaction system capable of providing a neural network training model in a supervised state, comprising: a storage unit for storing a plurality of pieces of data, a pre-training model resulting from training a predetermined neural network on the plurality of pieces of data, at least one first training program for the neural network, and at least one post-training model resulting from training with the at least one first training program on the plurality of pieces of data, wherein accuracy of the at least one post-training model is not equal to accuracy of the pre-training model; a supervision unit connected to the storage unit; a trade port connected to the supervision unit and enabling the supervision unit to be connected to an external third-party transaction platform; and a login port connected to the supervision unit and enabling a client to be connected via a cloud network, wherein the supervision unit receives a transaction information via the trade port and permits a specific client to download, in accordance with the transaction information, at least one of the pre-training model, the at least one first training program and the at least one post-training model from the storage unit via the login port.

Furthermore, the present disclosure further provides a cloud-based transaction method capable of providing a neural network training model in a supervised state, comprising the steps of: connecting a client to the login port via a cloud network; selecting, by the client, at least one of the pre-training model, the at least one first training program and the at least one post-training model, to purchase; paying, by the client, a fee of at least one of the pre-training model, the at least one first training program and the at least one post-training model to the third-party transaction platform, sending, by the third-party transaction platform, a transaction information to the supervision unit, giving, by the supervision unit, a download authority to the client according to the transaction information; and downloading, by the client, via the login port, and under supervision of the supervision unit, at least one of the pre-training model, the at least one first training program and the at least one post-training model, thus purchased.

Therefore, the present disclosure provides a graphical medical big data database accessible to the users, provides a pre-training model which results from training, and provides at least one training program whereby the users obtain a new training model, so as to enhance model accuracy.

Furthermore, the present disclosure further enables the users to upload additional training programs for carrying out training on the same data so as to obtain additional training models. Hence, in addition to the aforesaid pre-training model and post-training model, the present disclosure further allows the users to provide training programs for carrying out training on the aforesaid data so as to obtain new training models. Therefore, the present disclosure enhances the accuracy of the training models continually by enhancing the machine learning and deep learning functions of a neural network.

The client uploads a second training program for a neural network via the login port. The supervision unit permits the second training program to be stored in the storage unit. Training is carried out with the second training program on the plurality of pieces of data, allowing the cloud server to create an improvement training model and store the improvement training model in the storage unit.

### BRIEF DESCRIPTION OF THE SEVERAL VIEWS OF THE DRAWINGS

FIG. 1 is a block diagram of a cloud-based transaction system capable of providing a neural network training model in a supervised state according to the first preferred embodiment of the present disclosure.
FIG. 2 is a schematic view of the process flow of the operation of the cloud-based transaction system capable of providing a neural network training model in a supervised state according to the first preferred embodiment of the present disclosure.
FIG. 3 is another block diagram of the cloud-based transaction system capable of providing a neural network training model in a supervised state according to the first preferred embodiment of the present disclosure.
FIG. 4 is a block diagram of the cloud-based transaction system capable of providing a neural network training model in a supervised state according to the second preferred embodiment of the present disclosure.
FIG. 5 is a schematic view of the process flow of the operation of the cloud-based transaction system capable of providing a neural network training model in a supervised state according to the second preferred embodiment of the present disclosure.

### DETAILED DESCRIPTION OF THE INVENTION

Objectives, features, and advantages of the present disclosure are hereunder illustrated with preferred embodiments, depicted with drawings, and described below.

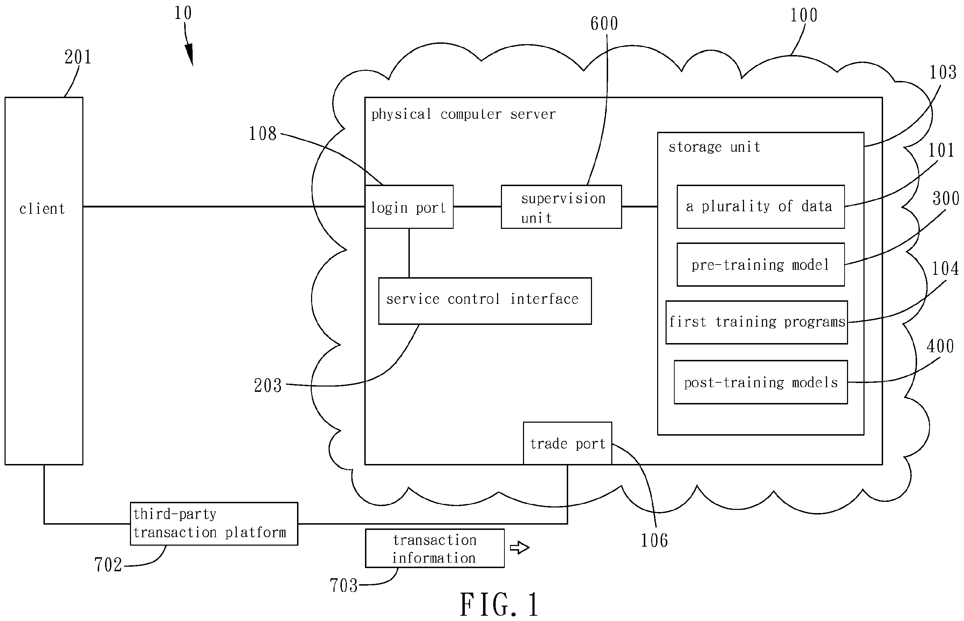

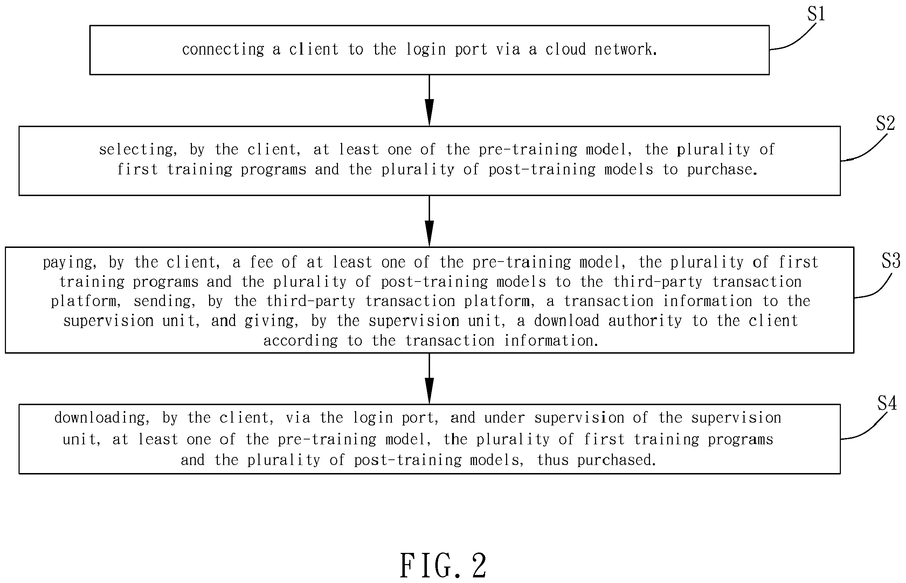

Referring to FIG. 1 and FIG. 2, a cloud-based transaction system 10 capable of providing a neural network training model in a supervised state according to the first preferred embodiment of the present disclosure essentially comprises a storage unit 103, a supervision unit 600, a trade port 106 and a login port 108.

The storage unit 103 stores a plurality of pieces of data 101, a pre-training model 300 which results from training a predetermined neural network (not shown) on the plurality of pieces of data 101, at least one first training program 104 for the neural network, and at least one post-training model 400 which results from training with the at least one first training program 104 on the plurality of pieces of data 101. The accuracy of the at least one post-training model 400 is not equal to the accuracy of the pre-training model 300. The predetermined neural network is a high-accuracy algorithmic training program developed by a manufacturer on its own but not published. The predetermined neural network and the at least one first training program 104 are Convolutional Neural Network (CNN), Recurrent Neural Network (RNN), eXtreme Gradient Boosting (XGBoost), Random Forest, Gradient Boosting Machine, or Support Vector Machine. The algorithm of the predetermined neural network differs from the algorithm of the at least one first training program 104; hence, the accuracy of the pre-training model 300 is not equal to the accuracy of the at least one post-training model 400. The plurality of pieces of data 101 is graphical medical data. The at least one first training program 104 is in the number of one or in the plural. In the case of the plurality of first training programs 104, in practice, each said first training program 104 has a unique algorithm according to the Convolutional Neural Network (CNN), Recurrent Neural Network (RNN), eXtreme Gradient Boosting (XGBoost), Random Forest, Gradient Boosting Machine, or Support Vector Machine, thereby rendering the first training programs 104 different from each other. The description below is exemplified by the plurality of first training programs 104 and thus exemplified by the plurality of post-training models 400, demonstrating variations in accuracy.

The supervision unit 600 is connected to the storage unit 103.

The trade port 106 is connected to the supervision unit 600. The trade port 106 enables the supervision unit 600 to be connected to an external third-party transaction platform 702.

The login port 108 is connected to the supervision unit 600. A client 201 is connected to the login port 108 via a cloud network. In practice, the storage unit 103, the supervision unit 600, the trade port 106 and the login port 108 are integrated into a cloud server 100. The cloud server 100 is a single physical computer server or a large-scale system which consists of physical computer servers. This embodiment is exemplified by one physical computer server capable of computation. In practice, the client 201 is a computer or a smartphone. Furthermore, in practice, after the client 201 has been connected to the login port 108, a service control interface 203 is operable by the client 201. The service control interface 203 is part of the system 10 (The system 10 further comprises the service control interface 203.) When the client 201 is connected to the login port 108, the client 201 can access the service control interface 203 via the login port 108, for example, the service control interface 203 is displayed in the form of a webpage on the client 201. Furthermore, in another embodiment, the service control interface 203 is a program installed on the client 201, and the service control interface 203 is displayed after the client 201 has been connected to the login port 108. The service control interface 203 offers the client 201 purchase options, namely the pre-training model 300, the first training programs 104 and the post-training models 400, to select from. Furthermore, the service control interface 203 provides a link to the third-party transaction platform 702 such that the client 201 can click the link to connect to the third-party transaction platform 702 with a view to paying for a selected one of the purchase options.

The supervision unit 600 receives, via the trade port 106, a transaction information 703 from the third-party transaction platform 702 and permits, in accordance with the transaction information 703, the client 201 to download at least one of the pre-training model 300, the plurality of first training programs 104 and the plurality of post-training models 400 from the storage unit 103 via the login port 108.

As shown in FIG. 2, in the first embodiment, a transaction method for conducting a transaction comprises the steps below.
S1: connecting the client 201 to the login port 108 via a cloud network.
S2: selecting, by the client 201 and with the service control interface 203, at least one of the pre-training model 300, the plurality of first training programs 104 and the plurality of post-training models 400 to purchase. The description below is exemplified by the client's purchasing all of the pre-training model, the plurality of first training programs and the plurality of post-training models.
S3: paying, by the client 201, a fee of at least one of the pre-training model 300, the plurality of first training programs 104 and the plurality of post-training models 400 to the third-party transaction platform 702, sending, by the third-party transaction platform 702, a transaction information 703 to the supervision unit 600, and giving, by the supervision unit 600, a download authority to the client 201 according to the transaction information 703.
S4: downloading, by the client 201, via the login port 108, and under supervision of the supervision unit 600, at least one of the pre-training model 300, the plurality of first training programs 104 and the plurality of post-training models 400, thus purchased.

By the time when step S4 is finished, the client 201 has already downloaded the purchased items under the supervision of the supervision unit 600. The accuracy of the pre-training model 300 is not equal to the accuracy of the plurality of post-training models 400, such that, upon completion of the aforesaid download process, the client 201 understands the difference in accuracy between the training models which result from training with different training programs, thereby enabling the client 201 to decide (in accordance with the difference in accuracy) whether to develop any training program of greater accuracy with a view to enhancing the accuracy of the training models. Hence, the present disclosure provides a graphical medical big data database for use by users, provides the pre-training model 300, and provides the plurality of first training programs 104 whereby the users access the plurality of post-training models 400, so as to enhance the accuracy of different training models, given the difference in accuracy between the different training models and the functional difference between deep learning and machine learning of the neural network of the plurality of first training programs 104. Furthermore, the existence of one and only one first training program 104 precludes any difference between the first training programs 104; hence, all the post-training models 400 result from training solely with the first training program 104 and thus do not have any difference therebetween. Therefore, it is only necessary to compare the accuracy of the post-training model 400 with the accuracy of the pre-training model 300.

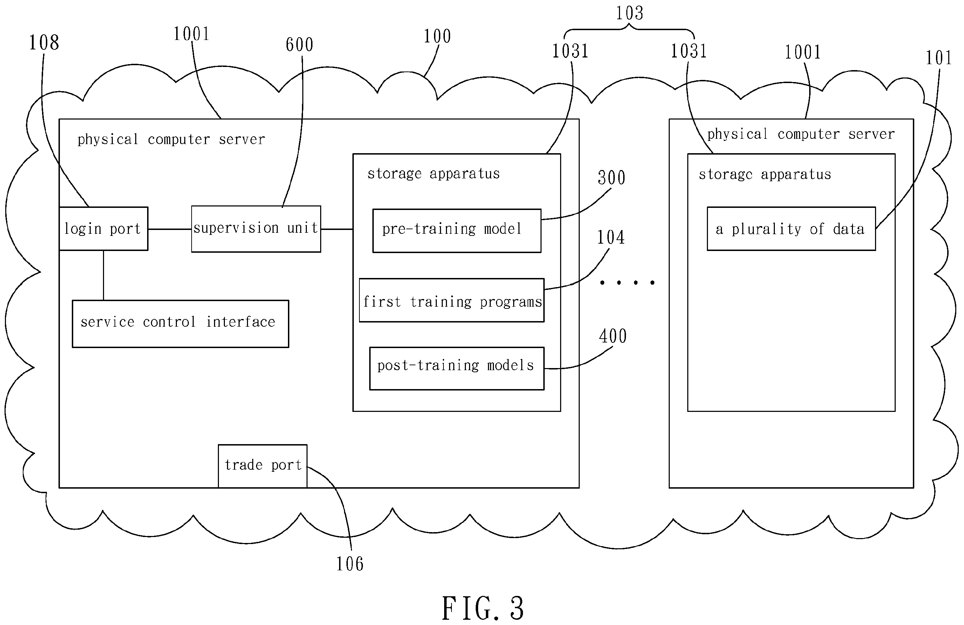

Furthermore, as shown in FIG. 3, if the storage unit 103, the supervision unit 600, the trade port 106 and the login port 108 are integrated into the system which comprises physical computer servers 1001, the storage unit 103 will comprise storage apparatuses 1031 contained in the physical computer servers 1001; hence, the pre-training model 300, the plurality of first training programs 104 and the plurality of post-training models 400 can be stored in the storage apparatus 1031 of one said physical computer server 1001, whereas only the physical computer server 1001 has the trade port 106 and the login port 108, allowing the plurality of pieces of data 101 to be stored in the storage apparatuses 1031 of the other physical computer servers 1001 in the system. Therefore, not only is storage implemented, but the plurality of pieces of data 101 is also not stored in the same storage apparatus 1031 together with the pre-training model 300, the plurality of first training programs 104 and the plurality of post-training models 400, so as to ensure that the users can download only the pre-training model 300, the plurality of first training programs 104 and the plurality of post-training models 400 but not the plurality of pieces of data 101, thereby providing extra protection for the plurality of pieces of data.

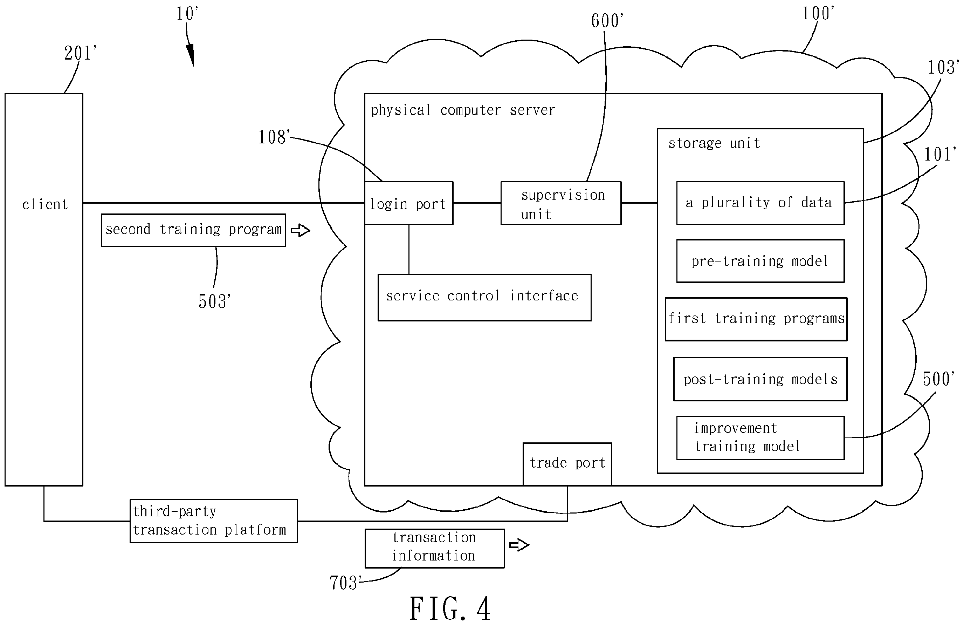

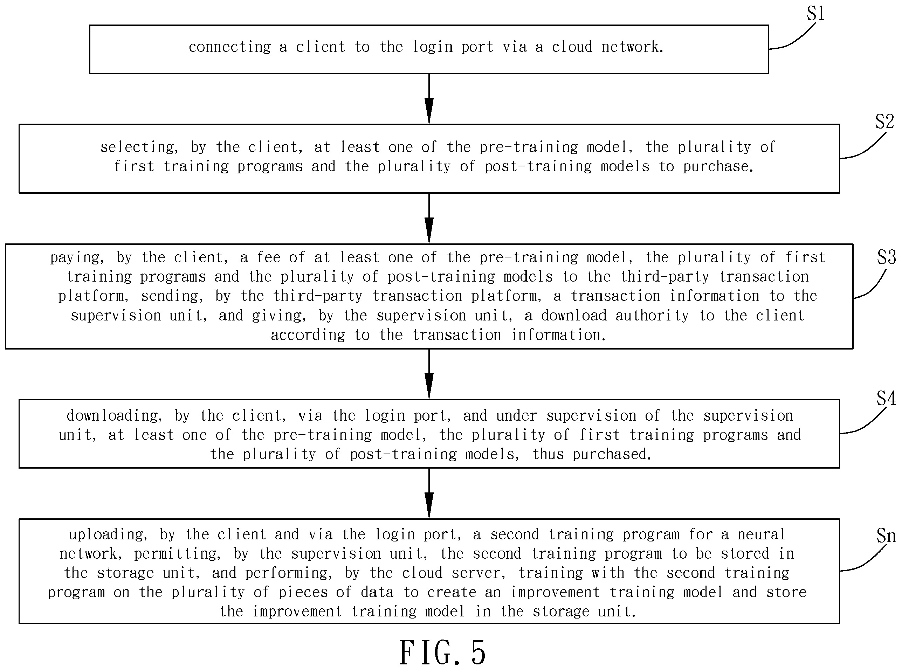

Referring to FIG. 4 and FIG. 5, the cloud-based transaction system 10' capable of providing a neural network training model in a supervised state according to the second preferred embodiment of the present disclosure is distinguished from the cloud-based transaction system 10 according to the first embodiment of the present disclosure by technical features described below.

In the second embodiment, the client 201' uses the login port 108' to upload the second training program 503' for a neural network. The supervision unit 600' permits the second training program 503' to be stored in the storage unit 103'. The cloud server 100' uses the second training program 503' to carry out training on the plurality of pieces of data 101' and thus create an improvement training model 500' and store the improvement training model 500' in the storage unit 103'. The cloud server 100' is a physical computer server capable of computation and thus able to perform the aforesaid training. With the second training program 503' being uploaded by the client 201' to the cloud server 100', the cloud server 100' permits the client 201' to download the improvement training model 500'; hence, in the absence of the transaction information 703', the supervision unit 600' only permits the client 201' to download, from the storage unit 103' and via the login port 108', the improvement training model 500' which results from training carried out with the second training program 503'.

In the second embodiment, the transaction method whereby the system 10' conducts a transaction not only comprises the aforesaid steps S1-S4 but also comprises a step Sn: uploading, by the client 201' and via the login port 108', the second training program 503', the supervision unit 600' permits the second training program 503' to be stored in the storage unit 103', and the cloud server 100' performs training with the second training program 503' on the plurality of pieces of data 101' to create the improvement training model 500' and store the improvement training model 500' in the storage unit 103'. Step Sn takes place after step S1, that is, before, behind or between steps S2-S4. For the sake of illustration, FIG. 5 depicts carrying out step Sn after step S4.

Therefore, the second embodiment not only allows the client 201' to download purchased items in a supervised state, obtain desired training models and understand the differences therebetween in accuracy, but also allows the client 201' to upload the second training program 503' which the client 201' favors and create the improvement training model 500' by carrying out training with the second training program 503'. Hence, the users can freely use the plurality of pieces of data 101' to further develop better training programs, thereby enhancing the accuracy of the training models greatly.

The other technical features and achievable advantages of the second embodiment are substantially identical to their counterparts of the first embodiment and thus are, for the sake of brevity, not described hereunder.

The plurality of pieces of data 101 of the present disclosure is medical image data collected by the Taiwan-based China Medical University & Hospital and dedicated to a clinical test scheme approved by the Taiwan-based China Medical University & Hospital Research Ethics Committee.

The above embodiments of the present disclosure are illustrative, rather than restrictive, of the present disclosure. Various modified designs made to the above embodiments according to the appended claims shall be deemed falling within the scope of the appended claims.

## Claims

1. A cloud-based transaction system (10)(10') capable of providing a neural network training model in a supervised state, comprising:
a storage unit (103)(103') for storing a plurality of pieces of data (101)(101'), a pre-training model (300) resulting from training a predetermined neural network on the plurality of pieces of data (101)(101'), at least one first training program (104) for the neural network, and at least one post-training model (400) resulting from training with the at least one first training program (104) on the plurality of pieces of data (101)(101'), wherein accuracy of the at least one post-training model (400) is not equal to accuracy of the pre-training model (300);
**characterized in that**
a supervision unit (600)(600') connected to the storage unit (103)(103');
a trade port (106) connected to the supervision unit (600)(600') and enabling the supervision unit (600)(600') to be connected to an external third-party transaction platform (702); and
a login port (108)(108') connected to the supervision unit (600)(600') and enabling a client (201)(201') to be connected via a cloud network,
wherein the supervision unit (600)(600') receives a transaction information (703)(703') via the trade port (106) and permits a specific client (201)(201') to download, in accordance with the transaction information (703)(703'), at least one of the pre-training model (300), the at least one first training program (104) and the at least one post-training model (400) from the storage unit (103)(103') via the login port (108)(108').

2. The cloud-based transaction system (10)(10') capable of providing a neural network training model in a supervised state according to claim 1, wherein the storage unit (103)(103'), the supervision unit (600)(600'), the trade port (106) and the login port (108)(108') are integrated into a cloud server (100)(100').

3. The cloud-based transaction system (10)(10') capable of providing a neural network training model in a supervised state according to claim 1, wherein the cloud server (100)(100') has at least one physical computer server (1001).

4. The cloud-based transaction system (10') capable of providing a neural network training model in a supervised state according to claim 2, wherein the client (201') uploads a second training program (503') for a neural network via the login port (108'), the supervision unit (600') permits the second training program (503') to be stored in the storage unit (103'), and the cloud server (100') carries out training with the second training program (503') on the plurality of pieces of data (101') to create an improvement training model (500') and store the improvement training model (500') in the storage unit (103').

5. The cloud-based transaction system (10') capable of providing a neural network training model in a supervised state according to claim 4, wherein, in absence of the transaction information (703'), the supervision unit (600') only permits the client (201') to download, from the storage unit (103') via the login port (108'), the improvement training model (500') which results from training carried out with the second training program (503').

6. The cloud-based transaction system (10)(10') capable of providing a neural network training model in a supervised state according to claim 1, further comprising a service control interface (203) operable by the client (201)(201') via the login port (108)(108') when the client (201)(201') is connected to the login port (108)(108'), wherein the service control interface (203) offers the client (201)(201') purchase options, namely the pre-training model (300), the at least one first training program (104) and the at least one post-training model (400), to select from, and the service control interface (203) provides a link to the third-party transaction platform (702).

7. A cloud-based transaction method capable of providing a neural network training model in a supervised state, using the system of claim 1, comprising steps of:
connecting a client (201)(201') to the login port (108)(108') via a cloud network;
selecting, by the client (201)(201'), at least one of the pre-training model (300), the at least one first training program (104) and the at least one post-training model (400), to purchase;
**characterized in that**
paying, by the client (201)(201'), a fee of at least one of the pre-training model (300), the at least one first training program (104) and the at least one post-training model (400) to the third-party transaction platform (702), sending, by the third-party transaction platform (702), a transaction information (703)(703') to the supervision unit (600)(600'), giving, by the supervision unit (600)(600'), a download authority to the client (201)(201') according to the transaction information (703)(703'); and
downloading, by the client (201)(201'), via the login port (108)(108'), and under supervision of the supervision unit (600)(600'), at least one of the pre-training model (300), the at least one first training program (104) and the at least one post-training model (400), thus purchased.

8. The cloud-based transaction method capable of providing a neural network training model in a supervised state according to claim 7, wherein the storage unit (103)(103'), the supervision unit (600)(600'), the trade port (106) and the login port (108)(108') are integrated into a cloud server (100)(100').

9. The cloud-based transaction method capable of providing a neural network training model in a supervised state according to claim 8, further comprising a step: uploading, by the client (201'), a second training program (503') for a neural network via the login port (108'), the supervision unit (600') permitting the second training program (503') to be stored in the storage unit (103'), and the cloud server (100') performing training with the second training program (503') on the plurality of pieces of data (101') so as to create an improvement training model (500') and store the improvement training model (500') in the storage unit (103').

10. The cloud-based transaction method capable of providing a neural network training model in a supervised state according to claim 9, wherein, in absence of the transaction information (703'), the supervision unit (600') only permits the client (201') to download, from the storage unit (103') and via the login port (108'), the improvement training model (500') which results from training carried out with the second training program (503').

11. The cloud-based transaction method capable of providing a neural network training model in a supervised state according to claim 7, further comprising a service control interface (203) operable by the client (201)(201') via the login port (108)(108') when the client (201)(201') is connected to the login port (108)(108'), wherein the service control interface (203) offers the client (201)(201') purchase options, namely the pre-training model (300), the at least one first training program (104) and the at least one post-training model (400), to select from, and the service control interface (203) provides a link to the third-party transaction platform (702).
